(19) Europäisches Patentamt
European Patent Office
Office européen des brevets

(11) Veröffentlichungsnummer: **0 275 901 B1**

# (12) EUROPÄISCHE PATENTSCHRIFT

(45) Veröffentlichungstag der Patentschrift: **09.06.93**

(21) Anmeldenummer: **88100349.5**

(22) Anmeldetag: **13.01.88**

(51) Int. Cl.5: **C12N 9/10**, C12N 1/20, C12P 35/00, //(C12N1/20, C12R1:06,1:125,1:37,1:38,1:39, 1:40),(C12N9/10,C12R1:06, 1:125,1:37,1:38,1:39,1:40)

## (54) Verwendung von Gamma-Glutamyl-Transpeptidase.

(30) Priorität: **17.01.87 DE 3701221**

(43) Veröffentlichungstag der Anmeldung:
**27.07.88 Patentblatt 88/30**

(45) Bekanntmachung des Hinweises auf die Patenterteilung:
**09.06.93 Patentblatt 93/23**

(84) Benannte Vertragsstaaten:
**AT BE CH DE ES FR GB GR IT LI LU NL SE**

(56) Entgegenhaltungen:

**ANN. MIRCROBIO. (INST. PASTEUR), Band 131A, 1980, Seiten 181-187, Paris, FR; G. GIAMMANCO et al.: "Intérét taxonomique de la recherche de la gamma-glutamyltransférase chez les enterobacteriaceae"**

(73) Patentinhaber: **HOECHST AKTIENGESELLSCHAFT**
**Postfach 80 03 20**
**W-6230 Frankfurt am Main 80(DE)**

(72) Erfinder: **Aretz, Werner, Dr.**
**Am Wäldchen 13**
**W-6240 Königstein/Taunus(DE)**
Erfinder: **Sauber, Klaus, Dr.**
**Friedrich-Ebert-Strasse 15**
**W-6231 Schwalbach am Taunus(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 275 901 B1

Rank Xerox (UK) Business Services
(3.10/3.6/3.3.1)

JOURNAL OF BACTERIOLOGY, Band 160, Nr. 1, Oktober 1984, Seiten 341-346, American Society for Microbiology, Washington, DC, US; R. NAKAYAMA et al.: "Purification and properties of gamma-glutamyltranspeptidase from Proteus mirabilis"

BIOCHEMICAL AND BIOPHYSICAL RESEARCH COMMUNICATIONS, Band 76, Nr. 2, 1977, Seiten 609-614, Academic Press, Inc., New York, NY, US; M.E. LEVITCH: "The demonstration of two discrete enzymes catalyzing the synthesis of glutamine and gamma-glutamylmethylamide in pseudomonas MS"

JOURNAL OF THE FACULTY OF AGRICULTURE KYUSHU UNIVERISTY, Band 30, Nr. 2-3, 1985, Seiten 95-105, Fukuoka, JP; T. HARA et al.: "Bacillus subtilis (natto) plasmid responsible for polyglutamate production encoding gamma-glutamyltranspeptidase"

## Beschreibung

γ-Glutamyl-Transpeptidasen (im folgenden γ-GTP) sind bereits aus Mikroorganismen isoliert worden [Ann. Microbiol. (Inst. Pasteur), 1980, 131 A, S. 181]. Sie spielen in den Mikroorganismen und in tierischen Geweben eine wichtige Rolle im Aminosäurestoffwechsel und im Glutathion-Zyklus [Meth. Enzymol. 77, 237 (1981)] und sind für den Transport verschiedener Aminosäuren in Form ihrer γ-Glutamyl-Derivate, die Bildung von Polyglutaminsäure in Bacilli sowie den Abbau von Glutathion (γ-Glutamyl-Cysteinyl-Glycin) verantwortlich.

Es wurde nun überraschend gefunden, daß einige Mikroorganismen γ-GTP synthetisieren, das zur Hydrolyse von Adipinyl- oder Glutaryl-Monoaminoverbindungenverwendet werden kann.

Die Erfindung betrifft somit

1. Die Verwendung von γ-Glutamyl-Transpeptidase (γ-GTP) erhältlich durch Fermentation von Bakterien und weiterhin gekennzeichnet durch
   - einen Molekulargewichtsbereich von 20.000 bis 60.000
   - einen isoelektrischen Punkt von pH 5,0 bis 6,5
   - ein pH-Optimum von 6,5 bis 10 bei L-γ-Glutamylparanitroanilid als Substrat
   - einen Km-Wert von 9 bis 36 μM bei pH 8

   zur enzymatischen Hydrolyse von Adipinyl- oder Glutaryl-Monoaminoverbindungen.

Im folgenden wird die Erfindung detailliert beschrieben, insbesondere in ihren bevorzugten Ausführungsformen. Weiterhin wird die Erfindung in den Patentansprüchen definiert.

γ-Glutamyltranspeptidase (γ-GTP) katalysiert die Hydrolyse von Glutaryl- oder Adipinyl-Monoaminoverbindungen mit beispielsweise der allgemeinen Formel,

$$R^1-(CH_2)_3-\underset{\underset{O}{\|}}{C}-NH-R^2$$

in der $R^1$ eine Carboxyl-, eine

$$\text{Carboxycarbonyl-}(-\overset{\overset{O}{\|}}{C}-COOH)-$$

oder eine Carboxymethylgruppe und $R^2$ Aminosäuren, Dipeptide, Cepheme, Cephame oder deren Derivate bedeutet, zu der entsprechenden Säure und der Monoaminoverbindung. Es werden bevorzugt 7-Aminocephalosporansäure-Derivate als Substrat eingesetzt, insbesondere α-Keto-adipinyl- oder Glutaryl-7-aminocephalosporansäure.

Das Enzympräparat kommt in Mikroorganismen periplasmatisch vor und läßt sich charakterisieren durch ein Molekulargewicht von 20.000 bis 60.000, bevorzugt 23.000 bis 40.000, insbesondere 30.000 bis 35.000 sowie durch einen isoelektrischen Punkt, der bei einem pH-Wert von 5,0 bis 6,5, bevorzugt 5,7 bis 6,1, liegt. Das pH-Optimum des Enzymprodukts liegt in dem pH-Bereich 6,5 bis 10 bei L-γ-Glutamylparanitroanilid als Substrat. Für das gleiche Substrat hat die erfindungsgemäße Transpeptidase einen Km-Wert von 9 bis 36 μM, bevorzugt 15 bis 20 μM, insbesondere 8,1 μM, bei pH 8.

In Gegenwart von Azaserin oder Jodacetamid wird γ-GTP irreversibel gehemmt. Bei Anwesenheit von Kupfer, Quecksilber und einer Mischung von Serin und Borat sowie in Gegenwart von 7-Aminocephalosporansäure zeigt das Enzym eine reversible Hemmung.

Die Herstellung erfolgt mit Hilfe von Mikroorganismen. In einem Screening wurden Bakterien, insbesondere die Gattungen Pseudomonas, Proteus, Arthrobacter und Bacillus gefunden, die γ-GTP in guten Ausbeuten liefern, bevorzugt geeignet sind beispielsweise:
Pseudomonas fragi DSM 3881 und Bacillus subtilis IFO 3025. Insbesondere bevorzugt wird das Enzym aus Pseudomonas fragi DSM 3881 gewonnen. Auch Mutanten und Varianten der genannten Mikroorganismen sind geeignet.

Die Anzucht der Mikroorganismen erfolgt aerob einzeln oder in Mischkultur beispielsweise submers unter Schütteln oder Rühren in Schüttelkolben oder Fermentern, gegebenenfalls unter Einführung von Luft oder Sauerstoff. Die Fermentation kann in einem Temperaturbereich von etwa 20 bis 37 °C, vorzugsweise

bei etwa 25 bis 30 °C, insbesondere bei 28 bis 30 °C, erfolgen. Es wird in einem pH-Bereich zwischen 5 und 8,5, vorzugsweise zwischen 5,5 und 8,0, fermentiert. Unter diesen Bedingungen zeigt die Kulturbrühe im allgemeinen nach 1 bis 3 Tagen eine nennenswerte Akkumulation des Enzyms. Die Synthese der γ-GTP beginnt in der späten log-Phase und erreicht ihr Maximum in der stationären Wachstumsphase. Die Produktion des periplasmatischen Enzyms kann mit Hilfe von Aktivitätstests durch HPLC-Analyse bzw. photometrisch verfolgt werden.

Die zur Produktion des γ-GTP verwendete Nährlösung enthält 0,2 bis 5 %, bevorzugt 0,5 bis 2 %, organische Stickstoffverbindungen sowie anorganische Salze. Als organische Stickstoffverbindungen kommen in Betracht:
Aminosäuren, Peptone, ferner Fleischextrakte, gemahlene Samen, beispielsweise von Mais, Weizen, Bohnen, Soja oder der Baumwollpflanze, Destillationsrückstände der Alkoholherstellung, Fleischmehle oder Hefeextrakte. An anorganischen Salzen kann die Nährlösung beispielsweise Chloride, Carbonate, Sulfate oder Phosphate der Alkali- oder Erdalkalimetalle, Eisen, Zink und Mangan enthalten, aber auch Ammoniumsalze und Nitrate.

Der Zusatz von assimilierbaren Kohlenhydraten steigert die Biomasseausbeute. Kohlenhydrate werden ebenfalls in den obengenannten Konzentrationen zugegeben. Als bevorzugte Kohlenstoffquelle können beispielsweise Zucker, wie Glucose oder Saccharose, sowie kohlenhydrathaltige Naturprodukte, wie Malzextrakt, der Nährlösung zugegeben werden.

Die optimalen Fermentations-Bedingungen sind für jeden Mikroorganismus zwar unterschiedlich aber entweder dem Fachmann schon bekannt oder aber in leichten Vorversuchen festzustellen.

Die Reinigung kann nach klassischen Verfahren über Lysozym-Aufschluß, Ammoniumsulfat-Fällung, Ionenaustauscher- und Gelpermeationschromatographie erfolgen. Die Kopplung des Enzyms ist nach gängigen Methoden möglich (Colowick und Kaplan, Meth. Enzymol., Vol. XLIV).

Für die enzymatische Umsetzung können sowohl ganze Zellen in freier oder immobilisierter Form unter Zusatz von β-Laktamase-Inaktivatoren, beispielsweise Clavulansäure oder Thienamycin, als auch das isolierte Enzymprodukt, das ebenfalls trägergebunden sein kann, eingesetzt werden. Geeignete Materialien für die Immobilisierung ganzer Zellen sind beispielsweise Chitosan, Alginat, χ-Carrageenan, Polyacrylhydrazide und weitere bekannte Stoffe aus literaturbekannten Verfahren (K. Venkatsubramanian, Immob. Cells (1979), ACS Symposium Series, S. 106).

γ-GTP hat insbesondere für die Gewinnung von 7-Aminocephalosporansäure aus Cephalosporin C technische Bedeutung, und zwar insbesondere seitdem eine Hefe bekannt geworden ist, nämlich Trigonopsis variabilis (DOS 2 219 454), mit deren Hilfe aus Cephalosporin C Glutaryl-7-aminocephalosporansaure in hohen Ausbeuten erzeugt werden kann. Diese Verbindung kann erfindungsgemäß mit γ-Glutamyl-Transpeptidase in guten Ausbeuten zu 7-Aminociphalosporansäure hydrolysiert werden. Damit ist eine Lücke im enzymatischen Abbau von Cephalosporin C zu der für die halbsynthetischen Cephalosporine technisch wichtigen 7-Aminocephalosporansäure geschlossen.

In den sich anschließenden Beispielen wird die Erfindung noch weitergehend beschrieben. Prozentangaben beziehen sich, wie auch in der vorangegangenen Beschreibung, auf das Gewicht, wenn nicht anders angegeben.

Beispiel 1

Die Stammhaltung der γ-GTP-produzierenden Mikroorganismen erfolgt auf Schrägagar folgender Zusammensetzung:

| | |
|---|---|
| Glucose | 1 % |
| Caseinpepton | 0,4 % |
| Fleischextrakt | 0,4 % |
| Hefeextrakt | 0,05 % |
| Leberextrakt | 0,05 % |
| NaCl | 0,25 % |
| pH 7,2 | |

Die Schrägröhrchen werden 2 Tage bei 28 °C bebrütet. Anschließend werden die Zellen mit 10 ml physiologischer Kochsalzlösung abgeschwemmt und 1 ml dieser Suspension zum Animpfen von 50 ml Vorkultur folgender Zusammensetzung in einem Erlenmeyerkolben mit 300 ml Fassungsvermögen verwendet:

| | |
|---|---|
| Pepton | 1 % |
| Malzextrakt | 0,5 % |
| pH 7,0 | |

Der Kolben wird bei 190 Upm auf einem Rotationsschüttler 24 Stunden bei 30 °C inkubiert. 2,5 ml dieser Kultur dienen als Inokulum von 50 ml Hauptkultur:

| A) Gram-negative Bakterien | | B) Bacilli | |
|---|---|---|---|
| Pepton | 1 % | Pepton | 0,12 % |
| Fleischextrakt | 0,5 % | Hefeextrakt | 0,12 % |
| NaCl | 0,5 % | Glucose | 0,25 % |
| $KH_2PO_4$ | 0,1 % | Na-Lactat (60 %) | 5,6 ml |
| $K_2HPO_4$ | 0,1 % | $NH_4Cl$ | 0,12 % |
| $MgSO_4 X 7H_2O$ | 0,05 % | $K_2HPO_4$ | 0,12 % |
| pH 7,0 | | $KH_2PO_4$ | 0,034 % |
| | | $MgSO_4$ x 7 $H_2O$ | 0,025 % |
| | | NaCl | 0,5 % |
| | | KCl | 0,5 % |
| | | $CaCl_2$ x 2 $H_2O$ | 0,0015 % |
| | | $MnCl_2$ x 4 $H_2O$ | 0,0007 % |
| | | $Fe(NH_4)$Citrat | 0,00015 % |

Die Kultur wird 24 Stunden bei 28 °C und einer Schüttelfrequenz von 190 Upm inkubiert und anschließend durch Zentrifugation abgeerntet.

In der folgenden Tabelle sind γ-GTP-Aktivitäten einiger Stämme aufgeführt.

| Stamm | γ-GTP (mU/ml Kulturlösung) |
|---|---|
| Ps. putida ATCC 17390 | 13 |
| Ps. aeruginosa NCTC 10701 | 11 |
| Proteus vulgaris ATCC 9634 | 26 |
| Ps. fragi DSM 3881 | 37 |
| B. subtilis IFO 3025 | 31 |

**Beispiel 2**

Analog Beispiel 1 wird eine Vorkultur mit Ps. fragi DSM 3881 angezogen. 50 ml dieser Kultur dienen als Inokulum für 2 l Hauptkulturlösung in eine 5 l-Fermenter. Die Anzucht des Stammes erfolgt bei 28 °C und einem Sauerstoffpartialdruck von 70 %. Die Bildung der γ-GTP wird photometrisch verfolgt und die Kultur bei maximalem Enzymtiter abgeerntet. Unter den gegebenen Bedingungen wird ein γ-GTP-Titer von 50 mU/ml Kulturlösung erreicht.

**Beispiel 3**

1 g der nach Beispiel 2 gewonnenen Zellen wird mit
2 ml Aufschlußpuffer folgender Zusammensetzung versetzt:
20 mM TRIS
10 mM EDTA pH 7,6
12 mM $MgSO_4$ x $7H_2O$

Nach dem Aufschlämmen werden folgenden Zusätze vorgenommen:
Lysozym: 0,8 mg/ml
DNAse: 10 mg/ml, davon 30 µl auf 1 ml Suspension
Es wird 15 Minuten bei Raumtemperatur inkubiert. Die lysierten Zellen werden bei 30000 g abgeschleudert. Der Überstand wird zu 20 Vol.-% mit einer 2 %igen Protaminsulfatlösung versetzt. Der Niederschlag wird nach Zentrifugation verworfen. Der Überstand wird einer fraktionierten Ammoniumsulfatfällung unterworfen (50 - 80 % der Sättigung). Das Pellet wird in 1/20 des ursprünglichen Volumens mit 0,1 M Citratpuffer, pH 5,0, wieder aufgenommen. Anschließend führt man eine Hitzbehandlung (1 h, 37 ° C) durch, zentrifugiert und dialysiert gegen 20 mM Citratpuffer, pH 5.0. Das Retentat wird auf eine Carboxymethylcellulose Säule aufgetragen. Es werden pro ml Enzymlösung 6 ml CM-Cellulose 52 von Whatman verwendet. Als Eluens benutzt man 20 mM Citratpuffer, pH 5,0, mit linearem Kochsalzgradienten (0 bis 0,5 M).

Die aktiven Fraktionen (HPLC-Test) werden gegen 10 mM TRIS-Puffer, pH 8,0, dialysiert und über eine DEAE-Cellulose-Säule (DE 52, Whatman) weiter gereinigt. Als Eluens dient 20 mM TRIS-Puffer, pH 8,0, mit linearem NaCl-Gradienten (0 bis 0,3 M).

Die aktivste Fraktion (Photometer-Test) dient für weitere Untersuchungen.

**Beispiel 4**

1 ml eines nach Beispiel 3 hergestellten Enzympräparats wird 5 : 1 konzentriert und auf eine Säule mit quervernetzter Agarose (®Superose 12, Pharmacia) gegeben. Als mobile Phase dient 50 mM Kaliumphosphatpuffer, pH 7,0 mit 0,15 M NaCl. Die Pumpgeschwindigkeit beträgt 0,3 ml pro Minute. Mit Hilfe von 15,4 ml Puffer wird die γ-Glutamyl-Transpeptidase eluiert. Sie kann mit L-γ-Glutamyl-p-$NO_2$-anilid und Spaltung von Glutaryl-7-Aminocephalosporansäure zu 7-Aminocephalosporansäure nachgewiesen werden. Die γ-GTP entspricht einem Molekulargewichtsbereich von 30.000 - 35.000.

**Beispiel 5**

Biomasse, die nach Beispiel 2 hergestellt worden ist, wird analog Beispiel 3 bis einschließlich der Hitzebehandlung aufgearbeitet. Dann wird gegen 20 mM Kaliumphosphatpuffer, pH 5,5, dialysiert; Zum Retentat wird soviel Carboxymethylcellulose (CM 52 von Whatman) gegeben, bis der Überstand Laktamase-frei ist. Nach Dialyse gegen 10 mM TRIS, pH 8,0, wird über eine DEAE-Cellulose-Säule (DE 52, Whatman) mit dem vierfachen Volumen chromatographiert. Als Eluens dient 20 mM TRIS-Puffer, pH 8,0, mit zusätzlich 0,1 M NaCl als linearer Gradient. Mit Hilfe des Photometer-Tests werden die aktiven Fraktionen ermittelt, wonach das Enzym mit 80 % Ammoniumsulfat ausgefällt wird. Das Pellet wird in 50 mM Kaliumphosphatpuffer, pH 7,0, und 0,15 M NaCl aufgenommen (25 : 1 konzentriert).

Eine weitere Reinigung erfolgt über eine Säule gefüllt mit einem Copolymer aus Dextran und Acrylamid (®Sephacryl, Pharmacia). Für 3,5 ml Konzentrat wird eine Säule mit 2,5 x 82 cm Abmessungen benutzt. Eluenspuffer ist 50 mM Kaliumphosphatpuffer, pH 7,0. Die mit dem HPLC-Test ermittelten aktiven Fraktionen werden mit Ammoniumsulfat konzentriert. Das Pellet wird in 20 mM Kaliumphosphatpuffer, pH 6,0, aufgenommen.

**Beispiel 6**

Für eine präparative Umsetzung von Glutaryl-7-Aminocephalosporansäure wird folgender Ansatz gewählt:
100 µl Enzymkonzentrat, hergestellt nach Beispiel 3, und
100 µl 40 mM Glutaryl-7-Aminocephalosporansäure, gelöst in 20 mM Kaliumphosphatpuffer, pH 6,0,
werden bei einer Temperatur von 33 ° C inkubiert.

Nach jeweils einer Stunde werden Aliquots entnommen und mit Hilfe der HPLC auf Bildung von 7-Aminocephalosporansäure untersucht. Nach ca. 2,5 Stunden sind 52 % des Ansatzes umgesetzt. Eine schrittweise pH-Erhöhung auf 7,0 erbringt einen maximalen Umsatz von 70 % nach insgesamt 8 Stunden.

**Beispiel 7**

Aktivitätsbestimmung von γ-GTP

a) HPLC-Test

50 µl 80 mM Glutaryl-7-Aminocephalosporansäure werden mit 100 bis 140 µl 250 mM Kaliumphosphatpuffer, pH 5,0, und 10 bis 50 µl Enzymlösung vermischt und bei 33 °C inkubiert. Nach jeweils 10 Minuten werden 20 µl Probe entnommen. Die Reaktion wird mit 20 µl Methanol abgestoppt. Es wird zentrifugiert und im Verhältnis 1 : 10 mit Wasser verdünnt. Eine Probe von 10 µl wird mittels HPLC auf den Gehalt an 7-Aminocephalosporansäure untersucht.
Stationäre Phase: C-18-Kieselgel
Mobile Phase: $KH_2PO_4$ 50 mM in $H_2O$, MeOH (80 : 20) + 0,001 % Tetrabutylammoniumsulfat

b) Photometrischer Test

600 µl L-γ-Glutamyl-p-nitroanilid (166 µM)
300 µl Kaliumphosphatpuffer, pH 5,7, 50 mM und
100 µl Kulturlösung werden miteinander vermischt und bei 37 °C inkubiert.

$$\epsilon_{405} = 9620 \frac{1}{\text{Mol}\cdot\text{cm}}$$

**Patentansprüche**

1. Verwendung von γ-Glutamyl-Transpeptidase (y-GTP) erhältlich durch Fermentation von Bakterien und weiterhin gekennzeichnet durch
   - einen Molekulargewichtsbereich von 20.000 bis 60.000
   - einen isoelektrischen Punkt von pH 5,0 bis 6,5
   - ein pH-Optimum von 6,5 bis 10 bei L-γ-Glutamylparanitroanilid als Substrat
   - einen Km-Wert von 9 bis 36 µM bei pH 8.

   zur enzymatischen Hydrolyse von Adipinyl- oder Glutaryl-Monoaminoverbindungen.

2. Verwendung von γ-GTP nach Anspruch 1 erhältlich durch Fermentation von Pseudomonas fragi DSM 3881 und/oder Bacillus subtilis IFO 3025.

3. Verwendung der γ-GTP nach Anspruch 1 oder 2 zur enzymatischen Hydrolyse von Adipinyl- oder Glutaryl-Monoaminoverbindungen.

4. Verwendung von γ-GTP nach Anspruch 1 oder 2 zur enzymatischen Hydrolyse von α-Keto-adipinyl- oder Glutaryl-7-aminocephalosporansäure.

**Claims**

1. The use of γ-glutamyltranspeptidase (γ-GTP) which can be obtained by fermentation of bacteria and, furthermore, has the following characteristics
   - a molecular weight range of 20,000 to 60,000
   - an isoelectric point of pH 5.0 to 6.5
   - a pH optimum of 6.5 to 10 with L-γ-glutamylparanitroanilide as substrate
   - a $K_m$ of 9 to 36 µM at pH 8,

   for the enzymatic hydrolysis of adipinyl- or glutaryl-monoamino compounds.

2. The use of γ-GTP as claimed in claim 1, obtainable by fermentation of Pseudomonas fragi DSM 3881 and/or Bacillus subtilis IFO 3025.

**3.** The use of the γ-GTP as claimed in claim 1 or 2 for the enzymatic hydrolysis of adipinyl- or glutaryl-monoamino compounds.

**4.** The use of γ-GTP as claimed in claim 1 or 2 for the enzymatic hydrolysis of α-keto-adipinyl- or glutaryl-7-aminocephalosporanic acid.

## Revendications

**1.** Utilisation de la γ-glutamyl-transpeptidase (γ-GTP) pouvant être obtenue par culture par fermentation de bactéries et en outre caractérisée par

- une plage de masse moléculaire allant de 20 000 à 60 000,
- un point isolélectrique allant de pH 5,0 à pH 6,5,
- un pH optimum de 6,5 à 10 dans le cas du L-γ-glutamylparanitroanilide en tant que substrat,
- une valeur $K_m$ de 9 à 36 μM à pH 8,

pour l'hydrolyse enzymatique de composés adipinyl- ou glutaryl-monoaminés.

**2.** Utilisation de la γ-GTP selon la revendication 1, pouvant être obtenue par culture par fermentation de Pseudomonas fragi DSM 3881 et/ou de Bacillus subtilis IFO 3025.

**3.** Utilisation de la γ-GTP selon la revendication 1 ou 2, pour l'hydrolyse enzymatique de composés adipinyl- ou glutaryl-monoaminés.

**4.** Utilisation de la γ-GTP selon la revendication 1 ou 2, pour l'hydrolyse enzymatique de l'acide α-céto-adipinyl- ou -glutaryl-7-aminocéphalosporanique.